# EUROPEAN PATENT APPLICATION

(11) **EP 1 591 114 A1**
(43) Date of publication of application: **02.11.2005**
(21) Application number: 04300137.9
(22) Date of filing: 12.03.2004
(51) Int. Cl.: A61K 31/155, A61K 31/337, A61P 3/04

(54) **Use of metformin and orlistat for the treatment or prevention of obesity**

(71) Applicant: Fournier Laboratories Ireland Limited, Carrigtwohill, Co. Cork (IE)
(72) Inventor: Junien, Jean-Louis, 92310 Sevres (FR); Edgar, Alan, 21490 Saint Julien (FR)
(74) Representative: Nevant, Marc

(57) **Abstract**

The present invention relates to the use of metformin and orlistat to treat patients suffering from obesity.

## Description

The present invention relates to the use of metformin and orlistat to treat patients suffering from obesity.

Tetrahydrolipstatin ("THL") is an inhibitor of pancreatic lipase and is known by the generic name orlistat. The use of THL as medicament, particularly as anti-obesity agent, and pharmaceutical compositions containing THL as active agent are described in U.S. Patent No. 4,598,089. A process for the preparation of orlistat is described in U.S. Patent No. 4,983,746. A pharmaceutical composition comprising orlistat and sibutramine is described in WO 99/33450.

Metformin is a biguanide that is mainly known for its antihyperglycaemic activity and is widely used in the treatment of non-insulin dependent diabetes; metformin can also be administered to the patient in combination with insulin.

It has now surprisingly been found that co-administration of metformin and orlistat results in beneficial effects in obese or overweight subjects.

Accordingly, the present invention relates a method for the treatment or prevention of obesity, comprising co-administering an effective dosage of metformin and orlistat.

In another embodiment, the invention includes a method for the treatment or prevention of obesity, comprising co-administering an effective dosage of metformin and orlistat, where the effective dosage of metformin is in the range of about 100 to about 3000 mg per day.

In another embodiment, the effective dosage of orlistat is in the range of about 50 to about 1440 mg per day.

In another embodiment, metformin and orlistat are administered simultaneously, in a method for the treatment or prevention of obesity, comprising co-administering an effective dosage of metformin and orlistat.

In another embodiment of a method for the treatment or prevention of obesity, metformin and orlistat are administered sequentially.

In another embodiment, the invention includes a method for the treatment or prevention of obesity in a patient already treated with orlistat, which comprises administering to the patient an effective dosage of metformin. As mentioned above, metformin and orlistat are administered simultaneously or sequentially.

In yet another embodiment, the invention includes a method for the treatment or prevention of obesity, further comprising co-administering an effective dosage of a fibrate and/or a statin. The fibrate and/or the statin can be administered simultaneously or sequentially.

In another embodiment, the invention includes the use of metformin, orlistat and a pharmaceutically acceptable carrier in the manufacture of a medicament for the treatment or prevention of obesity.

As demonstrated in the present specification, the use of metformin and orlistat, has led to favorably unexpected results in that body weight control is more efficient when orlistat is co-administered with metformin.

As used in this application, "co-administration" means the administration of two or more compounds to the same patient, within a time period of up to about three to about four hours. For example, co-administration encompasses (1) simultaneous administration of a first and second compound; (2) administration of a first compound, followed by administration of a second compound about 2 hours after administration of the first compound; and (3) administration of a first compound, followed by administration of a second compound about 4 hours after administration of the first compound. As described herein, the present invention encompasses co-administration of metformin and orlistat to a patient.

Metformin is commercially available e.g. as Glucophage® 850. Chemically, metformin is 1,1-dimethylbiguanide.

According to the invention, metformin can be administered as the free base or in the form of one of its pharmaceutically acceptable salts, such as the hydrochloride, acetate, benzoate, citrate, fumarate, embonate, chlorophenoxyacetate, glycolate, palmoate, aspartate, methanesulphonate, maleate, parachlorophenoxyisobutyrate, formate, lactate, succinate, sulphate, tartrate, cyclohexanecarboxylate, hexanoate, octonoate, decanoate, hexadecanoate, octodecanoate, benzenesulphonate, trimethoxybenzoate, paratoluenesulphonate, adamantanecarboxylate, glycoxylate, glutamate, pyrrolidonecarboxylate, naphthalenesulphonate, 1-glucosephosphate, nitrate, sulphite, dithionate or phosphate.

Among these salts, the hydrochloride, fumarate, embonate and chlorophenoxyacetate are more particularly preferred.

The pharmaceutically acceptable salts of metformin are obtained in a manner, which is known per se, by the action of metformin on the corresponding acid.

Orlistat is commercially available as Xenical® and is indicated in conjunction with a mildly hypocaloric diet for the treatment of obese patients with a body mass index (BMI) greater than or equal to 30 kg/m², or overweight patients (BMI ≥ 28 kg/m²) with associated risk factors.

Chemically, orlistat is [2S-[2α(R*),3β]]-N-formyl-L-leucine 1-[(3-hexyl-4-oxo-2-oxetanyl)methyl]dodecyl ester. It is also known as N-formyl-L-leucine ester with (3S,4S)-3-hexyl-4-[(2S)-2-hydroxy-tridecyl]-2-oxetanone, or (-)-tetrahydrolipstatin.

According to the present invention, a preparation is defined as the formulation of the active compound(s) with encapsulating material as a carrier providing a capsule in which the active component with or without other carriers, is surrounded by a carrier, which is thus in association with it. This includes tablets, powders, capsules, pills, cachets, and lozenges which can be used as solid dosage forms suitable for oral administration.

An effective dosage is defined in the present invention as the amount of a compound that prevents or ameliorates adverse conditions or symptoms of disease(s) or disorder(s) being treated. The amount to be administered to a patient and the frequency of administration to the subject can be readily determined by one of ordinary skill in the art by the use of known techniques and by observing results obtained under analogous circumstances. In determining the effective dosage, a number of factors are considered by the attending diagnostician, including but not limited to, the potency and duration of action of the compounds used; the nature and severity of the illness to be treated as well as on the sex, age weight, general health and individual responsiveness of the patient to be treated, and other relevant circumstances.

With respect to orlistat, the effective dosage is in the range of about 50 to about 1440 mg/day, preferably in the range of about 120 to about 720 mg/day and more preferably in the range of about 120 to about 360 mg/day, given in one or more doses, preferably three times daily. Orlistat is preferably administered orally, before the meals.

With respect to metformin, the effective dosage is in the range of about 100 mg to about 3000 mg/day, preferably in the range of about 500 mg to about 2550 mg/day, given in one or more doses, preferably two or three times daily. Metformin is preferably administered orally, during or at the end of the meals.

The present invention relates to the unexpected discovery that co-administration of metformin and orlistat exerts beneficial effects in overweight or obese subjects, i.e. subjects having a BMI ≥ 28 kg/m².

According to the invention, metformin and orlistat can be administered simultaneously, or sequentially. In a preferred embodiment of the invention, metformin and orlistat are administered simultaneously, more preferably in one formulation containing metformin and orlistat.

Thus, according to a another embodiment, the invention relates to a pharmaceutical composition containing metformin, orlistat and a pharmaceutically acceptable carrier. It will be understood that the composition contains a therapeutically effective amount of each active compound.

The expression "therapeutically effective" indicates the capability of a compound to prevent, or improve the severity of, the disorder, while avoiding adverse side effects typically associated with alternative therapies. The expression "therapeutically effective" is to be understood to be equivalent to the expression "effective for the treatment, prevention, or inhibition", and both are intended to qualify the amount of each compound for use in the combination therapy, which will achieve the goal of improvement in the control of body weight or treatment of obesity.

The relative amounts of each compound in the pharmaceutical composition may be varied and may be as described above. Metformin and orlistat that are described above can be provided in the pharmaceutical composition so that the preferred amounts of each compound are supplied by a single dosage form, for example a single injection or a single capsule, or by up to two, or more, dosage forms.

As described above, an embodiment of the present invention relates to a pharmaceutical composition comprising a therapeutically effective amount of a combination of metformin and orlistat and at least one pharmaceutically acceptable carrier, adjuvant or diluent and, if desired, other active compounds.

Thus, according to another embodiment, the pharmaceutical composition of the invention also comprises another active compound such as a fibrate and/or a statin.

Within the framework of the invention, fibrates are defined as PPARα agonists (peroxisome proliferator activated receptor alpha agonists), including fibric acid derivatives and pharmaceutically acceptable salts and esters of such fibric acid derivatives. Fibric acid derivatives lower the levels of triglyceride-rich lipoproteins, such as VLDL, raise HDL levels, and have variable effects on LDL levels. The effects on VLDL levels appear to result primarily from an increase in lipoprotein lipase activity, especially in muscle. This leads to enhanced hydrolysis of VLDL triglyceride content and enhanced VLDL catabolism. Fibric acid agents also may alter the composition of the VLDL, for example, by decreasing hepatic production of apoC-III, an inhibitor of lipoprotein lipase activity. These compounds are also reported to decrease hepatic VLDL triglyceride synthesis, possibly by inhibiting fatty acid synthesis and by promoting fatty acid oxidation.

The fibrate can be selected from the group consisting of gemfibrozil, fenofibrate, bezafibrate, clofibrate, ciprofibrate, beclobrate, binifibrate, ciplofibrate, clinofibrate, etofibrate, nicofibrate, pirifibrate, ronifibrate, simfibrate, theofibrate, a fibric acid derivative (e.g. fenofibric acid or clofibric acid) or a pharmaceutically acceptable salt or ester of said fibric acid derivative.

Preferably, the fibrate is fenofibrate, fenofibric acid or a pharmaceutically acceptable salt or ester of fenofibric acid.

In a preferred embodiment of the invention, the fibrate is fenofibrate. As used herein the term fenofibrate denotes (2-[4-(4-chlorobenzoyl) phenoxy]-2-methyl-propanoic acid, 1-methylethyl ester) or a salt thereof.

The fibrate can be of a reduced particle size, e.g. the fibrate particles have an average particle size of less than about 20 µm, preferably of less than about 10 µm. The fibrate can be micronised or co-micronised with a surfactant.

The effective dosage of the fibrate is in the range of about 10 to about 3000 mg/day given in one or more doses, preferably in the range of about 50 to about 1200 mg/day, and more preferably in the range of about 50 to about 300 mg/day. The skilled artisan will understand and appreciate that the effective dosage of a given fibrate will vary with the potency of the fibrate.

Within the framework of the invention, statins are defined as hydroxymethylglutaryl coenzyme A (HMG-CoA) reductase inhibitors. HMG-CoA reductase (3-hydroxy-3-methylglutaryl-coenzyme A) is the microsomal enzyme that catalyses the rate limiting reaction in cholesterol biosynthesis (Mevalonate). An HMG-CoA reductase inhibitor inhibits HMG-CoA reductase, and therefore inhibits or interferes with the synthesis of cholesterol. Inhibition of cholesterol synthesis can lead to a reduction in blood cholesterol levels.

The statin can be selected from the group consisting of lovastatin, fluvastatin, atorvastatin, simvastatin, pravastatin, itavastatin and rosuvastatin. The statin can be in the form of a salt selected from the group consisting of the chloride, bromide, iodide, sulfate, nitrate, phosphate, citrate, methanesulfonate, trifluoroacetate, acetate, sodium ion, potassium ion, magnesium ion, calcium ion, and an ammonium cation such as tetramethylammonium ion.

The effective dosage of the statin is in the range of about 0.1 mg to about 100 mg/day depending on the specific statin used.

The compositions of the invention are preferably administered enterally or parenterally (parenteral administration includes subcutaneous, intramuscular, intradermal, intramammary, intravenous, and other administrative methods known in the art), or better still orally, although the other routes of administration, for instance such as rectal administration, are not excluded.

For preparing oral pharmaceutical compositions from the compounds of this invention, inert, pharmaceutically acceptable carriers can be either solid or liquid. Solid form preparations include powders, tablets, coated tablets, dragees, troches, lozenges, dispersible granules, capsules, and sachets. Compositions for oral use may be prepared according to any method known in the art of manufacture of pharmaceutical compositions.

A solid carrier can be one or more substances which may also act as diluents, flavouring agents, solubilizers, lubricants, suspending agents, binders, or tablet disintegrating agents; it can also be an encapsulating material.

In powders, the carrier is a finely divided solid, which is in admixture with the finely divided active component. In tablets, the active compound is mixed with the carrier having the necessary binding properties in suitable proportions and compacted in the shape and size desired.

Suitable carriers include, for example, inert diluents, such as magnesium carbonate, calcium stearate, magnesium stearate, talc, lactose, sugar, pectin, dextrin, starch, tragacanth, methyl cellulose, sodium carboxymethyl cellulose, and the like.

The present invention also includes the formulation of metformin and orlistat with encapsulating material as a carrier providing a capsule in which metformin and orlistat (with or without other carriers) are surrounded by a carrier, which is thus in association with metformin and orlistat. In a similar manner, sachets are also included. Tablets, powders, sachets, and capsules can be used as solid dosage forms suitable for oral administration.

The tablets may be uncoated or coated by known techniques to delay disintegration and adsorption in the gastrointestinal tract and thereby provide a sustained action over a longer period. For example, a time delay material such as glyceryl monostearate or glyceryl distearate may be employed.

Formulations for oral use may also be presented as hard gelatin capsules wherein the active compounds are mixed with inert solid diluent, for example, calcium carbonate, calcium phosphate or kaolin, or as soft gelatin capsules wherein the active compounds are present as such, or mixed with water or an oil medium, for example, arachid oil, liquid paraffin, or olive oil.

Aqueous suspensions can be produced that contain the active compounds in admixture with excipients suitable for the manufacture of aqueous suspensions. Such excipients include suspending agents, such as sodium carboxymethylcellulose, methylcellulose, hydroxypropylmethylcellulose, sodium alginate, polyvinylpyrrolidone gum tragacanth and gum acacia; dispersing or wetting agents e.g. naturally-occuring phosphatides, such as lecithin, condensation products of an alkylene oxide with fatty acids, such as polyoxyethylene stearate, condensation products of an alkylene oxide with fatty acids, such as polyoxyethylene stearate, condensation products of ethylene oxide with long chain aliphatic alcohols, such as heptadecaethyleneoxycetanol, condensation products of ethylene oxide with partial esters derived from fatty acids and a hexitol, such as polyoxyethylene sorbitol monooleate, or condensation products of ethylene oxide with partial esters derived from fatty acids and hexitol anhydrides, such as polyoxyethylene sorbitan monooleate.

The aqueous suspensions may also contain one or more preservatives, for example, ethyl or n-propyl p-hydroxybenzoate, one or more colouring agents, one or more flavouring agents, one or more sweetening agents.

Oily suspensions may be formulated by suspending the active compounds in an omega-3 fatty acid, a vegetable oil, for example arachid oil, olive oil, sesame oil or coconut oil, or in a mineral oil such as liquid paraffin. The oily suspensions may contain a thickening agent, for example, beeswax, hard paraffin or cetyl alcohol.

Sweetening agents and flavouring agents may be added to provide a palatable oral preparation, which may be preserved by the addition of an antioxydant such as ascorbic acid.

Dispersible powders and granules suitable for the preparation of an aqueous suspension by the addition of water provide the active compounds in admixture with a dispersing or wetting agent, a suspending agent and one or more preservatives. Suitable dispersing or wetting agents and suspending agents are exemplified by those already mentioned above. Additional excipients, for example sweetening, flavouring and colouring agents, may also be present.

Syrups and elixirs containing the novel combination may be formulated with sweetening agents. Such formulations may also contain a demulcent, a preservative and flavouring and colouring agents.

Liquid form preparations include solutions, suspensions and emulsions suitable for oral administration. Aqueous solutions for oral administration can be prepared by dissolving the active compounds in water and adding suitable flavouring agents, colouring agents, stabilizers, and thickening agents as desired. Ethanol, propylene glycol and other pharmaceutically acceptable non-aqueous solvents may be added to improve the solubility of the active compounds. Aqueous suspensions for oral use can be made by dispersing the finely divided active compounds in water together with a viscous material such as natural or synthetic gums, resins, methyl cellulose, sodium carboxymethyl cellulose, and other suspending agents known in the pharmaceutical formulation art.

Preferably, the pharmaceutical composition is in unit dosage form. In such form, the preparation is divided into unit doses containing appropriate amounts of the active compounds. The unit dosage form can be a packaged preparation, the package containing discrete amounts of the preparation, for example, packeted tablets, capsules, and powders in vials or ampoules. The unit dosage form can also be a capsule, cachet, or tablet itself, or it can be the appropriate number of any of these packaged forms.

Formulations developed for metformin can be used for the pharmaceutical composition of the invention containing metformin and orlistat. Such formulations of metformin are described in the following patents: gastric retentive (WO 9855107 and WO 9907342), controlled-release metformin composition (WO 0236100), controlled-release with unitary core (WO 9947125), treatment with 400 mg or below of metformin (US 6,100,300), novel salts of metformin (WO 9929314), biphasic controlled-release delivery system (WO 9947128), metformin preparation (WO 9608243), controlled-release (WO 0103964 and WO 0239984), metformin tablet (WO 03004009), sustained-release composition (WO 02067905), controlled-release composition (WO 0211701), gastroretentive (WO 0006129), solid carriers for improved delivery (WO 0137808), coating for sustained-release composition (WO 02085335), modified-release composition (WO 03002151), liquid formulation of metformin (WO 0247607), controlled-release device (WO 02094227), metformin quick release tablet (JP 2002326927). Among these formulations, the metformin once a day formulation is preferred.

The compositions of the invention can also be administered parenterally either subcutaneously, or intravenously, or intramuscularly, or intrasternally, or by infusion techniques, in the form of sterile injectable aqueous or olagenous suspensions. Such suspensions may be formulated according to the known art using those suitable dispersing or wetting agents and suspending agents which have been mentioned above, or other acceptable agents. The sterile injectable preparation may also be a sterile injectable solution or suspension in a non-toxic parenterally acceptable diluent or solvent, for example as a solution in 1,3-butanediol. Among the acceptable vehicles and solvent that may be employed, water, Ringer's solution and isotonic sodium chloride solution may be mentioned. In addition, sterile, fixed oils are conventionally employed as a solvent or suspending medium. For this purpose, any bland fixed oil may be employed including synthetic mono- or diglycerides. In addition, n-3 polyunsaturated fatty acids may find use in the preparation of injectables.

The compositions of the invention can also be administered by inhalation, in the form of aerosols or solutions for nebulizers, or rectally in the form of suppositories prepared by mixing the drug with a suitable non irritating excipient, which is solid at ordinary temperature but liquid at the rectal temperature and will therefore melt in the rectum to release the drug. Such materials include cocoa butter and polyethylene glycols.

Preferably, the composition is a controlled-release composition.

Daily dosages can vary within wide limits and will be adjusted to the individual requirements in each particular case. In general, for administration to adults, an appropriate daily dosage has been described above, although the limits that were identified as being preferred may be exceeded if expedient. The daily dosage can be administered as a single or divided dose.

The amount of each compound to be administered will depend on a number of factors including the age of the patient, the severity of the condition and the past medical history of the patient. Each unit dose generally contains (1) from about 100 to 1000 mg of metformin and/or (2) from about 50 to about 720 mg, preferably about 120 to about 360 mg, of orlistat. Typical unit doses preferably contain 500 mg, 850 mg or 1000 mg of metformin (850 mg being especially preferred) and/or 120 mg of orlistat. It will however be appreciated that formulations containing doses of metformin and/or orlistat which are bioequivalent to the preferred doses mentioned above, are within the scope of the invention. For example, a formulation containing a dose of metformin which is bioequivalent to the dose of the Glucophage ® 850 formulation, is encompassed by the appended claims.

When the pharmaceutical composition comprises a fibrate, each unit dose generally contains from about 10 to about 1000 mg, preferably about 50 to 600 mg, more preferably about 50 to about 200 mg, of fibrate. When the pharmaceutical composition comprises a statin, each unit dose generally contains from about 0.1 to 100 mg of statin, e.g. 0.1, 0.3, 0.8, 1, 2, 5, 10, 20, 40 or 80 mg of statin.

The present invention further relates to kits that are suitable for use in performing the methods of treatment described above. In one embodiment, the kit contains (i) one or more unit doses of metformin, (ii) one or more unit doses of orlistat, (iii) optionally one or more unit doses of a fibrate, and (iv) optionally one or more unit doses of a statin, for a simultaneous or sequential administration, in amounts sufficient to carry out the methods of the present invention.

The invention is illustrated by the following example, which is not to be construed as limiting, but merely as an illustration of some preferred features of the invention.

### EXAMPLE: Effect of metformin and orlistat co-administration on body weight

This study was designed to evaluate the effects of a combination of metformin and orlistat on body weight.

The data from this study, which are summarized in Table 1, demonstrate that (1) there is a significant difference between mice treated with metformin alone or with orlistat alone, and mice fed with a standard diet (no normalization of body weight is observed in treated mice), while (2) there is no significant difference between mice treated with a combination of metformin and orlistat, and mice fed with a standard diet (the body weight of treated mice is normalized).

Therefore, a better control of the body weight is obtained when orlistat is administered in combination with metformin.

### METHOD

*Animals:* C57BL/6 EOPS male mice, weighing approximately 20 g, were used in the study. They were put by 5 into individual cages in a temperature-, humidity-and light-controlled room (21-23°C ± 2 °C, 12h-12h light-dark cycle). They were fed with either a standard laboratory diet or a high-fat diet, and had free access to water. After acclimatization, they were randomized into groups of 20, based on body weight.

The experimental groups were:
- Group 1: mice fed with standard diet
- Group 2: mice fed with high-fat diet, treated with orlistat 2 mg/kg p.o., mixed with the diet
- Group 3: mice fed with high-fat diet, treated with metformin 100 mg/kg p.o., mixed with the diet
- Group 4: mice fed with high-fat diet, treated with orlistat 2 mg/kg p.o. and metformin 100 mg/kg p.o., mixed with the diet.

Body weight was recorded at the beginning of the study, and after one and three weeks of treatment, respectively.

*Statistics :* All data are presented as mean ± s.e.m. Results were subjected to covariance analysis corrected from baseline followed by Turkey-Kramer adjustment for multiple comparisons. A p < 0.1 was considered significant (vs. standard diet).

**Table 1**

| **Treatment** | **Body weight gain (g)** **after 1 week** | **Body weight gain (g)** **after 3 weeks** |
|---|---|---|
| Standard diet | +0.23 ± 0.12 | + 2.1 ± 0.19 |
| Orlistat, 2mg/kg | + 3.2 ± 0.48*** | + 5.5 ± 0.63*** |
| Metformin, 100 mg/kg | + 1.3 ± 0.14** | + 3.5 ± 0.25* |
| Orlistat 2 mg/kg + Metformin 100 mg/kg | + 0.99 ± 0.14° | + 2.8 ± 0.21° |

| | | |
|---|---|---|
| * p < 0.1; | | |
| **p < 0.05; | | |
| *** p < 0.0001; | | |
| ° = not significant | | |

## Claims

1. Use of metformin, orlistat and a pharmaceutically acceptable carrier in the manufacture of a medicament for the treatment of obesity.

2. The use according to claim 1, wherein metformin is in the form of a phannaceutically acceptable salt.

3. The use according to claim 2, wherein said salt is selected from the group consisiting of the hydrochloride, acetate, benzoate, citrate, fumarate, embonate, chlorophenoxyacetate, glycolate, palmoate, aspartate, methanesulphonate, maleate, parachlorophenoxyisobutyrate, formate, lactate, succinate, sulphate, tartrate, cyclohexanecarboxylate, hexanoate, octonoate, decanoate, hexadecanoate, octodecanoate, benzenesulphonate, trimethoxybenzoate, paratoluenesulphonate, adamantanecarboxylate, glycoxylate, glutamate, pyrrolidonecarboxylate, naphthalenesulphonate, 1-glucosephosphate, nitrate, sulphite, dithionate and phosphate.

4. The use according to one of claims 1 to 3, wherein metformin is in the form of a pharmaceutically acceptable salt which is selected from the group consisting of the hydrochloride, fumarate, embonate, and chlorophenoxyacetate.

5. The use according to one of claims 1 to 4, wherein metformin and orlistat are administered simultaneously or sequentially.

6. The use according to one of claims 1 to 5 wherein the effective dosage of metformin is in the range of about 100 to about 3000 mg per day.

7. The use according to one of claims 1 to 6, wherein the effective dosage of orlistat is in the range of about 50 to about 1440 mg per day.

8. A pharmaceutical composition containing metformin, orlistat and a pharmaceutically acceptable carrier.

9. The pharmaceutical composition according to claim 8, wherein metformin is in the form of a pharmaceutically acceptable salt.

10. The pharmaceutical composition according to claim 9, wherein said salt is selected from the group consisiting of the hydrochloride, acetate, benzoate, citrate, fumarate, embonate, chlorophenoxyacetate, glycolate, palmoate, aspartate, methanesulphonate, maleate, parachlorophenoxyisobutyrate, formate, lactate, succinate, sulphate, tartrate, cyclohexanecarboxylate, hexanoate, octonoate, decanoate, hexadecanoate, octodecanoate, benzenesulphonate, trimethoxybenzoate, paratoluenesulphonate, adamantanecarboxylate, glycoxylate, glutamate, pyrrolidonecarboxylate, naphthalenesulphonate, 1-glucosephosphate, nitrate, sulphite, dithionate and phosphate.

11. The pharmaceutical composition according to one of claims 8 to 10, wherein metformin is in the form of a pharmaceutically acceptable salt which is selected from the group consisting of the hydrochloride, fumarate, embonate, and chlorophenoxyacetate.

12. The pharmaceutical composition according to one of claims 8 to 11, containing from about 100 to about 1000 mg of metformin.

13. The pharmaceutical composition according to one of claims 8 to 12, containing from about 50 to about 720 mg of orlistat.

14. The pharmaceutical composition according to one of claims 8 to 13, containing 500 mg, 850 mg or 1000 mg of metformin and 120 mg of orlistat.
